# FASCICULE DE BREVET EUROPEEN

(11) **EP 1 815 800 B1**
(45) Date de publication et mention de la délivrance du brevet: **07.07.2010**
(21) Numéro de dépôt: 06024129.6
(22) Date de dépôt: 14.09.1998
(51) Int. Cl.: A61B 17/00

(54) **Instrument chirurgical de fixation de tissu mou**
Chirurgisches Instrument zum Fixieren von weichem Gewebe
Surgical instrument for fixing soft tissue

(30) Priorité: 12.09.1997 FR 9711600
(43) Date de publication de la demande: 08.08.2007
(62) Demande divisionnaire de: 98943979.9
(73) Titulaire: Evalve, Inc., Menlo Park, CA 94025 (US)
(72) Inventeur: Seguin, Jacques, 75009 Paris (FR)
(74) Mandataire: Clark, Jane Anne

(56) Documents cités:
- EP-A- 0 558 031
- WO-A-81/00668
- WO-A-94/18893
- DE-C- 3 504 292
- GB-A- 2 151 142

## Description

La présente invention concerne un instrument chirurgical permettant, par voie percutanée, de fixer l'une à l'autre deux zones de tissu mou, normalement mutuellement distantes. Cet instrument est en particulier destiné à la "reconstruction" de valves cardiaques, notamment la valve mitrale, ou au traitement de toute malformation d'une cloison cardiaque.

Une pathologie de la valve mitrale d'un coeur, dite "insuffisance mitrale", consiste dans le fait que la valve ne se ferme pas correctement, c'est-à-dire de manière étanche, et donc qu'elle ne joue plus son rôle de clapet anti-reflux entre l'oreillette gauche et le ventricule gauche.

Il est alors nécessaire de procéder à une chirurgie de réparation de cette valve. Selon la technique actuelle, le sternum est scié (sternotomie) et la cage thoracique est ouverte, pour aborder le coeur. Le patient est alors mis en circulation sanguine extra-corporelle et, le coeur étant arrêté, les cavités cardiaques sont ouvertes pour accéder directement à la valve mitrale, en général par l'oreillette gauche.

Un traitement adéquat de cette valve est alors opéré, notamment par annuloplastie ou, plus récemment, par suture du bord libre de la valvule dite "antérieure" au bord libre de la valvule dite "postérieure" dans la zone correspondant à l'insuffisance mitrale.

Cette technique opératoire présente les inconvénients d'être particulièrement complexe et difficile à mettre en oeuvre, de nécessiter une anesthésie générale du patient, une sternotomie et un arrêt du coeur relayé par une circulation extra-corporelle. Cette dernière implique l'utilisation d'anti-coagulants à hautes doses au cours de l'intervention. En outre, cette technique induit un risque opératoire certain, en particulier un risqua d'infarctus du myocarde et d'hémorragie.

EP 0558031 divulgue un appareil chirurgical servant à fixer l'une à l'autre deux portions de tissu cutané corporel, qui se compose de : une paire d'organes allongés opposés munis de mâchoires qui peuvent se rapprocher l'un de l'autre dans une position de rapprochement mutuel, afin d'engager deux portions de tissu corporel positionnés au sein des membres, et une pluralité d'attaches amovibles semblables à une tige pour pénétrer dans les tissus corporels en vue de fixer les portions de tissu corporel entre elles.

WO 94/18893 divulgue un rétracteur et un dissecteur à utiliser chez un patient, possédant un ou plusieurs organes articulés aptes à tenir et à tirer le tissu.

La présente invention vise à remédier à ces inconvénients en fournissant un instrument chirurgical permettant, d'une manière générale, de fixer par voie percutanée deux zones de tissu mou normalement mutuellement distantes, et, dans l'application particulière précitée, de fixer une zone du bord libre de la valvule mitrale antérieure à une zone du bord libre de la valvule mitrale postérieure.

Selon la présente invention, il est fourni un instrument tel que celui décrit à la revendication 1.

L'instrument selon l'invention permet ainsi, par voie percutanée, de saisir, de rapprocher et de fixer les deux zones de tissu, simplement en manipulant, depuis l'extérieur du corps du patient, les différents organes que comprend cet instrument.

De préférence, le tube, les organes allongés et la ou les tiges précitées présentent une souplesse telle que l'instrument peut être introduit, par voie percutanée, jusqu'à l'intérieur d'un coeur pour permettre le traitement des valvules d'une valve cardiaque, notamment la valve mitrale ; chacun desdits organes allongés est réalisé en un matériau présentant une souplesse élastique, et comprend une portion d'extrémité distale qui diverge normalement de l'axe longitudinal de l'organe allongé ; les deux organes allongés sont mobiles axialement par rapport au tube, entre une position de rétractation dans ce tube, dans laquelle lesdites portions d'extrémité distales sont déformées élastiquement de telle sorte que les extrémités distales desdits organes sont mutuellement rapprochées, et une position d'extension, dans laquelle ces portions d'extrémité distales retrouvent leur forme neutre et divergent par conséquent l'une de l'autre, de manière à venir à proximité des tissus, en vue de la saisie de ceux-ci.

Avantageusement, les organes allongés comprennent des moyens permettant de rendre leurs extrémités distales soit actives, en vue de la saisie des zones de tissu, soit inactives, pour empêcher cette saisie et faciliter ainsi l'introduction, le déplacement ou le retrait de l'instrument.

Chaque organe allongé peut être constitué par une tige en matériau élastique, à extrémité distale recourbée et/ou conformée en harpon, et par une gaine engagée et pouvant coulisser axialement sur cette tige, cette gaine pouvant, dans une position avancée, recouvrir ladite extrémité distale et être reculée par rapport à cette extrémité distale, pour, dans une position reculée, découvrir celle-ci.

Selon une variante de réalisation, chaque organe allongé peut être constitué par un tube relié à un dispositif de mise du volume interne de ce tube en dépression. Cette mise en dépression permet de saisir la zone de tissu correspondante, tandis que la mise à l'air libre de ce volume permet de lâcher le tissu, sans lésion de celui-ci. L'extrémité distale de l'organe allongé présente avantageusement dans ce cas une forme évasés, assurant une surface de saisie de dimensions suffisantes.

Selon une variante de réalisation l'instrument comprend deux organes d'accrochage, dont un premier est positionné de manière à être fixé du côté distal des tissus et dont le deuxième, destiné à être fixé du côté proximal des tissus, est situé entre ledit premier organe d'accrochage et l'organe formant butée.

Les deux organes d'accrochage peuvent ainsi être placés de part et d'autre des tissus à réunir et être pressés contre ledit organe de butée, pour assurer la parfaite fixation des tissus.

Pour sa bonne compréhension, l'invention est à nouveau décrite ci-dessous en référence au dessin schématique annexé représentant, à titre d'exemples non limitatifs, deux formes de réalisation préférées de l'instrument qu'elle concerne.

La figure 1 est une vue en coupe longitudinale d'un coeur dont la valve mitrale ne se ferme pas correctement et doit être traitée au moyen de cet instrument ;
la figure 2 est une vue en plan de la valve mitrale avant traitement ;
la figure 3 est une vue d'une valve mitrale similaire à la figure 2, après traitement par suture, selon la technique classique ;
la figure 4 est une vue similaire à la figure 1 d'un coeur dans lequel l'instrument selon l'invention a été introduit ;
la figure 5 est une vue de l'extrémité distale de l'instrument, à échelle agrandie et en coupe longitudinale ;
la figure 6 est une vue de cette extrémité distale, selon la ligne VI-VI de la figure 5 ;
les figures 7 et 8 sont des vues similaires à la figure 5, dans deux phases différentes d'utilisation de l'instrument ;
la figure 9 est une vue similaire à la figure 2 de la valve mitrale après le traitement opéré au moyen dudit instrument ;
la figure 10 est une vue de cette valve selon la ligne X-X de la figure 9, et
la figure 11 est une vue similaire à la figure 5 de l'instrument selon une deuxième forme de réalisation.

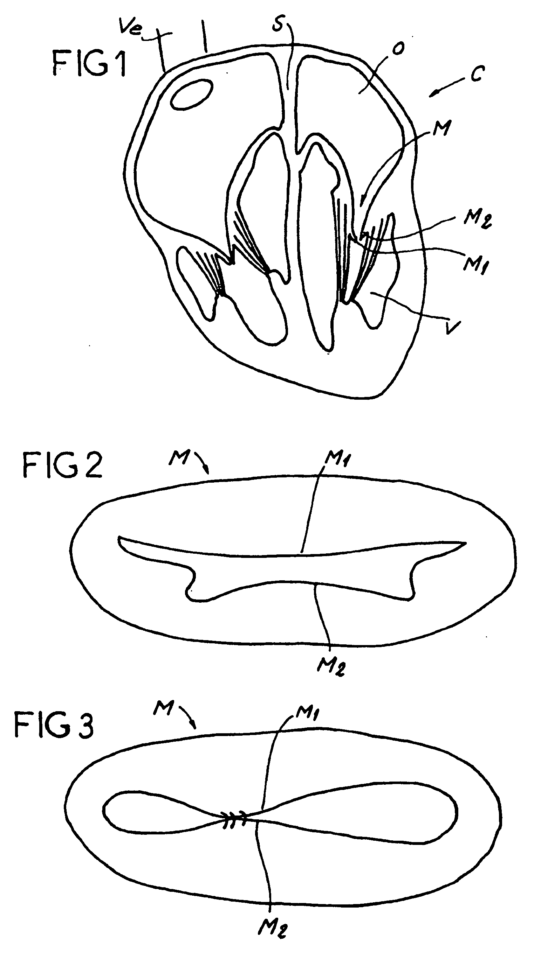

La figure 1 montre un coeur C dont la valve mitrale M présente une mauvaise coaptation des valvules M1, M2, de telle sorte que cette valve M ne se ferme pas correctement, c'est-à-dire de manière étanche, et qu'elle ne joue plus son rôle de clapet anti-retour entre l'oreillette gauche 0 et le ventricule gauche V.

Selon une technique de traitement classique, après sternotomie et ouverture de la cage thoracique, le patient est mis en circulation sanguine extra-corporelle. Le coeur est arrêté et les cavités cardiaques sont ouvertes pour aborder directement la valve M afin de suturer les bords libres opposés des deux valvules M1, M2, comme montré aux figures 2 et 3.

Cette fixation des valvules M1, M2 l'une à l'autre permet de redonner une coaptation satisfaisante de ces valvules M1, M2, et donc de restaurer l'étanchéité de la valve M.

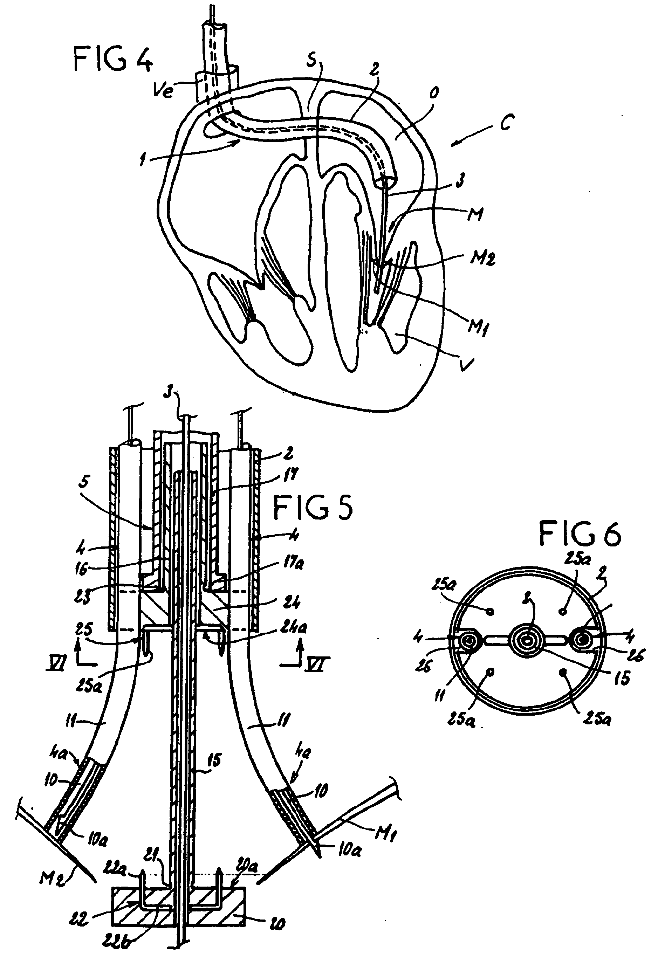

Les figures 4 à 8 montrent un instrument 1 permettant d'opérer par voie percutanée une telle fixation des valvules M1, M2 l'une à l'autre.

Cet instrument 1 comprend un tube extérieur 2, un fil axial de guidage 3, deux organes allongés 4 et un dispositif d'agrafage 5. L'ensemble présente une souplesse suffisante pour pouvoir être introduit, par voie percutanée, jusqu'à l'intérieur du coeur C, par la veine cave Ve et au travers du septum interauriculaire S, de telle sorte que le fil 3 soit engagé au travers de la valve M et que l'extrémité distale du tube 2 se trouve située dans l'oreillette gauche 0, en regard de cette valve M.

Chacun des organes allongés 4 présente une portion d'extrémité distale 4a ayant normalement une forme courbe, et comprend une tige 10 en matériau relativement rigide mais élastique, notamment en métal, et une gaine 11, notamment en matériau synthétique.

L'extrémité distale 10a de la tige 10 est acérée et est plus ou moins conformée en harpon.

La gaine 11 est engagée sur la tige 10 et peut coulisser par rapport à elle de telle sorte que, dans une position avancée, montrée sur la gauche de la figure 5, elle recouvre l'extrémité 10a et, dans une position reculée, montrée sur la droite de la figure 5, elle découvre cette même extrémité 10a.

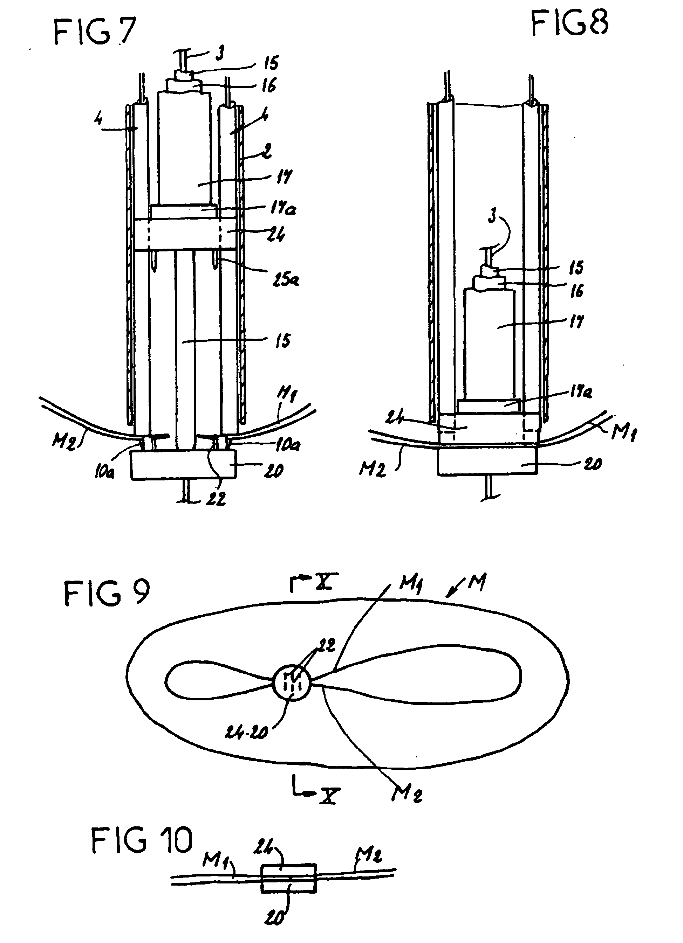

Les organes allongés 4 ont une longueur telle qu'ils dépassent au-delà de l'extrémité proximale du tube 2. Ils peuvent ainsi être déplacés axialement par rapport au tube 2 entre une position de rétractation, permettant un rapprochement mutuel des extrémités 10a (figures 7 et 8) et une position d'extension, permettant un éloignement mutuel de ces mêmes extrémités 10a (figure 5).

Le dispositif d'agrafage 5 comprend trois tiges tubulaires concentriques 15, 16, 17 engagés sur le fil 3, pouvant coulisser par rapport à celui-ci et les uns par rapport aux autres.

La tige intérieure 15 est reliée à un disque 20 par une zone sécable 21, l'ensemble étant en matière synthétique moulée. Ce disque 20 est percé axialement pour permettre le passage du fil 3 et comprend une agrafe 22. Les branches latérales 22a de cette agrafe 22 font saillie de la face proximale 20a du disque 20 et la branche centrale 22b, comprenant un anneau central pour le passage du fil 3, est noyée dans la matière constituant le disque 20.

La tige intermédiaire 16 est reliée, également par une zone sécable 23, à un disque 24 comprenant deux agrafes 25. Les branches latérales 25a de ces agrafes font saillie de la face distale 24a de ce disque 24 et leurs branches centrales sont noyées dans la matière constituant le disque 24.

Ce dernier comprend en outre deux encoches latérales 26 diamétralement opposées, pour permettre le passage des organes allongés 4.

La tige extérieure 17 présente, quant à elle, une extrémité distale élargie 17a propre à venir en appui contre la face proximale du disque 24.

Chacune de ces tiges 15, 16, 17 fait saillie au-delà de 1'extrémité proximale du tube 2, de manière à pouvoir être déplacée par l'opérateur. Des moyens de positionnement, de déplacement relatif et de traction de ces tiges, tels que des poignées ou des systèmes à vis-écrous, sont prévus sur les extrémités proximales de ces tiges, pour permettre d'opérer une traction sur la tige 15 tout en maintenant la tige 17 dans une position déterminée par rapport au tube 2, puis sur la tige 16 tout en maintenant également la tige 17 dans une position déterminée par rapport au tube 2.

En pratique, le fil 3 est tout d'abord introduit à travers la veine cave Ve, le septum interauriculaire S puis la valve mitrale M, sous contrôle échographique ou radioscopique, puis le tube 2 et les différents organes qu'il contient sont engagés dans la veine Ve, au travers du septum S, jusqu'à ce que l'extrémité distale du tube 2 se trouve en regard de la valve mitrale M.

Au moment de cette introduction, le disque 20 se trouve maintenu sensiblement à la hauteur de l'ouverture du tube 2, qu'il permet de clore, les organes 4 étant rétractés de telle sorte que leurs extrémités distales soient situées dans les encoches 26.

Lorsque l'extrémité distale du tube 2 est en position adéquate, la tige 15 est déplacée pour amener le disque 20 au-delà des valvules M1, M2, puis les organes 4 sont coulissés vers leur position d'extension, comme montré à la figure 5. Ces organes 4 se déploient alors par élasticité, de telle sorte que leurs portions d'extrémité distales 4a s'éloignent l'une de l'autre. Les extrémités distales 10a des tiges 10 se trouvent alors à proximité des valvules M1, M2.

Les gaines 11 sont alors reculées par rapport aux tiges 10, pour découvrir les extrémités 10a, puis ces dernières sont fichées chacune dans la valvule M1 ou M2 correspondante.

Le tube 2 est alors avancé vers la valve mitrale M, ce qui a pour effet, ainsi que le montre la figure 7, de rapprocher les portions 4a l'une de l'autre, et donc de rapprocher les bords libres des deux valvules M1, M2.

Une traction est opérée sur la tige 15 en maintenant la tige 17 en position par rapport au tube 2, afin successivement de faire pénétrer les branches 22a et 25a des agrafes 22, 25 dans les valvules M1, M2, de déformer ces branches 22a et 25a contre les parois respectives en regard 20a, 24a des disques 20, 24, et de rompre la zone sécable 21. Cette rupture correspond à une déformation suffisante des branches des agrafes pour assurer une parfaite fixation mutuelle des valvules M1, M2.

Les gaines 11 sont ensuite déplacées par rapport aux tiges 10 pour venir en appui contre les valvules M1, M2, afin de permettre la séparation facile des extrémités 10a et des valvules M1, M2, puis une traction est opérée sur la tige 16 tout en maintenant la tige 17 en position, de manière à rompre la zone 23.

Les valvules M1, M2 se trouvent alors agrafées l'une à l'autre par leur bord libre, ainsi que cela apparaît aux figures 9 et 10.

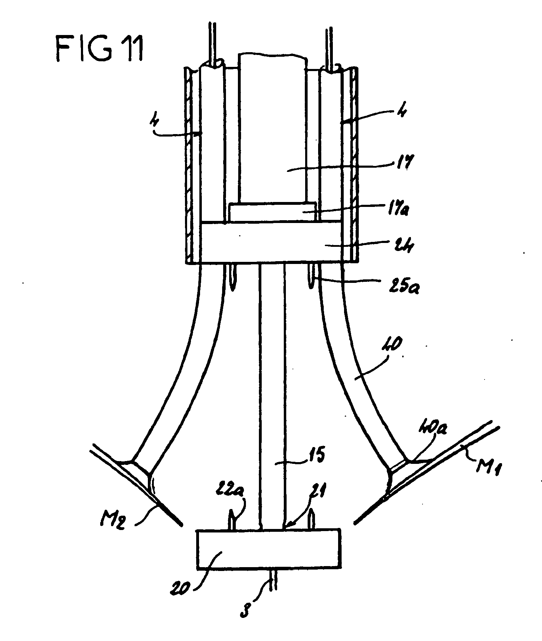

La figure 11 montre une variante de réalisation dans laquelle les tiges 10 et gaines 11 sont remplacées par deux cathéters 40, dont les extrémités distales 40a sont évasées. Ces cathéters 40 dépassent de l'extrémité proximale du tube 2 et sont reliés à des seringues de mise de leur volume interne en dépression. La saisie ou le relâchement des valvules M1, M2 s'opère alors respectivement par mise en dépression ou en surpression du volume interne des cathéters 40. Les extrémités évasées 40a permettent d'assurer une surface de préhension suffisante sur les valvules M1, M2. Elles sont de structure souple de manière à pouvoir être engagées, en étant légèrement repliées, entre la paroi du tube 2 et deux pans coupés latéraux du disque 20. Pour le reste, les autres éléments de cet instrument sont similaires à ceux déjà décrits, et sont désignés par les mêmes références numériques.

Il va de soi que l'invention n'est pas limitée à la forme de réalisation décrite ci-dessus à titre d'exemple. Ainsi, les extrémités 10a peuvent être recourbées en forme de "J" dans leur forme neutre, pour crocheter les tissus, les gaines 11 permettant alors, lorsqu'elles recouvrent ces extrémités, de les déformer pour les maintenir dans un état non recourbées ; les organes 4 et les moyens de fixation peuvent être placés dans des tubes séparés ; l'instrument 1 peut être introduit par voie tant veineuse qu'artérielle.

## Revendications

1. Instrument (1) chirurgical permettant, par voie percutanée, de fixer l'une à l'autre deux zones de tissu mou, normalement mutuellement distantes, comprenant
- au moins un tube (2) pouvant être introduit par voie percutanée dans le corps du patient jusqu'à ce que son extrémité distale soit située à proximité des deux zones de tissu à réunir ;
- deux organes allongés (4) engagés dans ce tube, dont chacun comprend une extrémité distale (10a) propre à saisir l'une des deux zones de tissu à réunir, lesdits organes allongés étant déplaçables vers une position d'extension dans laquelle les deux extrémités distales s'étendent au-delà de ladite extrémité distale dudit tube ;
- des moyens permettant de déplacer les portions d'extrémité distales (4a) de ces organes allongés entre une position de rapprochement mutuel, dans laquelle ces portions ne font pas obstacle à l'introduction du tube et permettent, après saisie, de rapprocher lesdites zones de tissu, et une position d'éloignement mutuel, dans laquelle chacune desdites extrémités distales est apte à saisir la zone correspondante de tissu ;
- au moins un organe d'accrochage (22,25), muni de moyens d'accrochage (22a,25a) pour chacun des tissus à réunir, cet organe d'accrochage permettant de fixer les deux zones de tissu lorsqu'elles sont rapprochées l'une de l'autre par le déplacement desdites portions d'extrémité distales dans une position de rapprochement mutuel ;
- une tige (15,16) reliée à chaque organe d'accrochage et pouvant être manipulée depuis l'extérieur du corps du patient pour déplacer axialement ledit organe d'accrochage, cette tige étant séparable de cet organe d'accrochage ; et
- un organe (17a) formant butée, contenu dans le ou les tubes, permettant d'immobiliser axialement chaque organe d'accrochage lorsqu'une traction est opérée sur la dite tige de manière à presser l'organe d'accrochage contre ledit organe formant butée, jusqu'à réaliser la séparation de la tige et de l'organe d'accrochage.

## Claims

1. A surgical instrument (1) enabling two zones of soft tissue that are normally mutually distant to be percutaneously joined together, comprising:
- at least one tube (2) that can be introduced percutaneously into the body of the patient until its distal extremity is situated in the proximity of the two zones of tissue to be joined;
- two elongated members (4) arranged in the tube, of which each comprises a distal extremity (10a) suitable for gripping one of the two tissue zones to be joined, said elongated members being movable towards an extended position in which their distal extremities (4a) extend beyond said distal extremity of said tube;
- means for displacing the distal end portions of the elongated members between a mutually close position, in which said portions do not hinder the introduction of the tube and enabling said tissue zones, once gripped, to be brought together, and a mutually distant position, in which each of said distal extremities is able to grip the corresponding tissue zone;
- at least one coupling member (22, 25), provided with coupling means (22a, 25a) for each tissue zone to be joined, the coupling member enabling the two tissue zones to be fixed to each other once brought together by the displacement of said distal end portions to said mutually close position;
- a stem (15, 16) linked to each coupling member, and which can be manipulated from outside the patient's body to displace axially said coupling member, said stem being separable from the coupling member, and
- an abutment forming member (17a), contained in the tube or tubes, enabling axial immobilisation of each coupling member when traction is applied to said stem so as to press the coupling member against said abutment forming member, until separation of the stem from the coupling member is achieved.

## Patentansprüche

1. Chirurgisches Instrument (1), das es ermöglicht, auf perkutanem Weg zwei Bereiche eines weichen Gewebes aneinander zu befestigen, die normalerweise voneinander beabstandet sind, das aufweist,
- zumindest ein Rohr (2), das in den Körper des Patienten über einen perkutanen Weg eingeführt werden kann, bis sein distales Ende in der Nähe der zwei Bereiche des Gewebes platziert ist, die zusammengebracht werden;
- zwei verlängerte Einrichtungen (4), die in das Rohr eingesetzt sind, von denen jede ein distales Ende (10a) aufweist, das eingerichtet ist, um einen der zwei Bereiche des zusammenzubringenden Gewebes zu greifen, wobei die verlängerten Einrichtungen in eine erstreckte Position versetzbar sind, in der die zwei distalen Enden sich über die des distalen Endes des Rohres hinaus erstrecken;
- Mittel, die es ermöglichen, die Abschnitte (4a) an den distalen Enden der verlängerten Einrichtungen zwischen einer Position des gegenseitigen Zusammenbringens, in der die Abschnitte kein Hindernis gegen das Einführen des Rohres sind, und es erlauben, nach dem Ergreifen, die gesamten Bereiche des Gewebes zusammenzubringen, und einer gegenseitig beabstandeten Position zu versetzen, in der jedes der distalen Enden dazu geeignet ist, um den entsprechenden Bereich des Gewebes zu ergreifen;
- zumindest eine Befestigungseinrichtung (22, 25), die mit Mitteln zum Befestigen (22a, 25a) für jedes der Gewebe, die zusammenzubringen sind, ausgestattet ist, wobei es jede Befestigungseinrichtung ermöglicht, die zwei Bereiche des Gewebes festzulegen, bis sie eines gegen das andere durch Versetzung der distalen Endabschnitte in eine gegeneinander zusammengebrachte Stellung zusammengebracht sind;
- ein Gestänge, das mit jeder der Befestigungseinrichtungen verbunden ist, und von dem Äußeren des Körpers des Patienten manipuliert werden kann, um die Befestigungseinrichtung axial zu versetzen, wobei dieses Gestänge von der Befestigungseinrichtung trennbar ist; und
- eine Einrichtung (17a), die einen Anschlag ausbildet, die sich in das oder die Rohre fortsetzt, die es ermöglicht, jede Befestigungseinrichtung axial festzusetzen, wenn ein Zug auf das Gestänge in einer Weise ausgeübt wird, um die Befestigungseinrichtung gegen die Einrichtung zu drücken, die den Anschlag bildet, bis die Trennung des Gestänges und der Befestigungseinrichtung durchgeführt ist.
